(19) Europäisches Patentamt
European Patent Office
Office européen des brevets

(11) **EP 3 539 039 B1**

(12) **EUROPEAN PATENT SPECIFICATION**

(45) Date of publication and mention
of the grant of the patent:
**24.12.2025 Bulletin 2025/52**

(21) Application number: **17811755.2**

(22) Date of filing: **09.11.2017**

(51) International Patent Classification (IPC):
**C12Q 1/6869** (2018.01)    **G16B 5/20** (2019.01)
**G16B 30/10** (2019.01)

(52) Cooperative Patent Classification (CPC):
(C-Sets available)
**G16B 30/00; C12Q 1/6869; G16B 5/00; G16B 5/20;
G16B 30/10; G16B 40/00**    (Cont.)

(86) International application number:
**PCT/US2017/060752**

(87) International publication number:
**WO 2018/089567 (17.05.2018 Gazette 2018/20)**

(54) **METHODS, SYSTEMS AND COMPUTER READABLE MEDIA TO CORRECT BASE CALLS IN REPEAT REGIONS OF NUCLEIC ACID SEQUENCE READS**

VERFAHREN, SYSTEM UND COMPUTER LESBARES MEDIUM ZUR KORREKTUR VON LESEFEHLERN IN REPEAT REGIONEN VON NUKLEINSAURESEQUENZ-READS

PROCÉDÉS, SYSTÈMES ET SUPPORTS LISIBLES PAR ORDINATEUR POUR CORRIGER DES APPELS DE BASE DANS DES RÉGIONS DE RÉPÉTITION DE LECTURES DE SÉQUENCE D'ACIDE NUCLÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2016  US 201662420022 P**

(43) Date of publication of application:
**18.09.2019  Bulletin 2019/38**

(73) Proprietor: **Life Technologies Corporation
Carlsbad, CA 92008 (US)**

(72) Inventor: **EL-DIFRAWY, Sameh
Carlsbad, CA 92008 (US)**

(74) Representative: **Thermo Fisher Scientific-
Life Sciences Solutions Group
Life Technologies
Frankfurter Straße 129b
64293 Darmstadt (DE)**

(56) References cited:
WO-A1-2016/038220    US-A1- 2013 345 066
US-A1- 2014 163 900    US-A1- 2014 316 716
US-A1- 2015 178 446

- PEREIRA M S ET AL: "Statistical learning formulation of the DNA base-calling problem and its solution in a Bayesian EM framework", DISCRETE APPLIED MATHEMATICS ELSEVIER NETHERLANDS, vol. 104, no. 1-3, 15 August 2000 (2000-08-15), pages 229 - 258, XP002778046, ISSN: 0166-218X
- ANDY S. ALIC ET AL: "Objective review of de novo stand-alone error correction methods for NGS data : Objective review of de novo stand-alone NGS correctors", WILEY INTERDISCIPLINARY REVIEWS: COMPUTATIONAL MOLECULAR SCIENCE, vol. 6, no. 2, 11 January 2016 (2016-01-11), pages 111 - 146, XP055449573, ISSN: 1759-0876, DOI: 10.1002/wcms.1239

• **ADRIANTO WIRAWAN ET AL: "HECTOR: a parallel multistage homopolymer spectrum based error corrector for 454 sequencing data",** BMC BIOINFORMATICS, BIOMED CENTRAL, LONDON, GB, vol. 15, no. 1, 6 May 2014 (2014-05-06), pages 131, XP021185561, ISSN: 1471-2105, DOI: 10.1186/1471-2105-15-131

(52) Cooperative Patent Classification (CPC): (Cont.)

C-Sets
**C12Q 1/6869, C12Q 2535/122, C12Q 2537/165**

**Description**

**CROSS-REFERENCE**

This application claims the benefit under 35 U.S.C. § 119(e) of U.S. Provisional Application no. 62/420,022, filed November 10, 2016.

**BRIEF SUMMARY OF THE INVENTION**

Accurately determining the length of a short tandem repeat (STR) region is an important method for human identification (HID) and other applications. Longer STR sequences may have a negative impact on the quality of the sequencing process that increases homopolymer errors in the repeat sequence. The increased homopolymer errors can generate false alleles and make it difficult to compare DNA profiles to a data base or identify mixtures of DNA where there are alleles from different individuals in a sample. US2013/0345066 discloses systems and methods for identifying sequence variations taking account of homopolymers in a flow space.

Variations such as a Single Nucleotide Polymorphism (SNP), insertion and deletion may exist in the flanks adjacent to the STR region. Detecting variations in the flanks associating those variations with the length of the STR can increase the discrimination power of the STR analysis.

According to an exemplary embodiment, there is provided a method of nucleic acid sequence analysis, including: (1) receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases; (2) for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region; (3) receiving a plurality of flow space signal measurements corresponding to the set of repeat region sequences; (4) determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and (5) modifying a base call at the position in the repeat region sequence to the homopolymer length associated with the optimum cluster for the flow space signal measurements for the given flow to produce a corrected repeat region sequence, thereby correcting an insertion error or a deletion error.

According to an exemplary embodiment, there is provided a system for nucleic acid sequence analysis, including a processor configured to perform the steps including: (1) receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases; (2) for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region; (3) receiving a plurality of flow space signal measurements corresponding to the set of repeat region sequences; (4) determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and (5) modifying a base call at the position in the repeat region sequence to the homopolymer length associated with the optimum cluster for the flow space signal measurements for the given flow to produce a corrected repeat region sequence, thereby correcting an insertion error or a deletion error.

According to an exemplary embodiment, there is provided a non-transitory machine-readable storage medium comprising instructions which, when executed by a processor, cause the processor to perform a method for nucleic acid sequence analysis, including: (1) receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases; (2) for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region; (3) receiving a plurality of flow space signal measurements corresponding to the set of repeat region sequences; (4) determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and (5) modifying a base call at the position in the repeat region sequence to the homopolymer length associated with the optimum cluster for the flow space signal measurements for the given flow to produce a corrected repeat region sequence, thereby correcting an insertion error or a deletion error.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 illustrates an example of a sequence read having an STR region.
FIG. 2 illustrates an example of alignment of left and right flank regions of a sequence read with those of a reference sequence.
FIG. 3 is an example of error behavior when the repeat sequence of bases includes a 3-mer or 4-mer homopolymer.
FIG. 4 is a block diagram of a method of nucleic acid sequence analysis to correct base calls in repeat region sequences, according to an exemplary embodiment.
FIG. 5 shows an example of a flow space histogram of the flow space signal measurements for one flow and where the repeat sequence includes 2-mer homopolymer.
FIG. 6 shows an example of a flow space histogram of the flow space signal measurements for one flow, where the repeat sequence includes 2-mer homopolymer and where two alleles are present.
FIG. 7A shows an example of a base space histogram of base calls versus homopolymer length, where the base calls were made for signal measurements at a single flow.
FIG. 7B shows an example of the flow space histogram for the same signal measurements corresponding the base space histogram of FIG. 7A.
FIG. 8A shows an example of a histogram of the number of repeats, where the repeat region sequences resulted from base calling.
FIG. 8B shows an example of a histogram of the number of repeats where the repeat region sequences include base correction, in accordance with an exemplary embodiment.
FIG. 9A shows an example of a histogram of the number of repeats where the repeat region sequences resulted from base calling.
FIG. 9B shows an example of a histogram of the number of repeats where the repeat region sequences include base correction, in accordance with an exemplary embodiment.
FIG. 10A shows an example of a histogram of the number of repeats, where the repeat region sequences resulted from base calling.
FIG. 10B shows an example of a histogram of the number of repeats where the repeat region sequences include base correction, in accordance with an exemplary embodiment.
FIG. 11 is a block diagram of a method of nucleic acid sequence analysis to detect variants in flanks of repeat region sequences, according to an exemplary embodiment.
FIG. 12 illustrates an example of a variant in a right flank of an STR region.
FIG. 13 shows an exemplary representation of flow space signal measurements from which base calls may be made.
FIG. 14 is a schematic diagram of an exemplary system for reconstructing a nucleic acid sequence, in accordance with various embodiments.

## DETAILED DESCRIPTION OF THE INVENTION

In accordance with the teachings and principles embodied in this application, new computer implemented methods, systems and non-transitory machine-readable storage medium are provided to mitigate insertion errors and deletion errors in STR sequences and improve accuracy in determination of the number of repeats. Further teachings provide for detecting variations in the flanks associating those variations with the length of the STR to improve the discrimination capability of STR analysis.

In various embodiments, DNA (deoxyribonucleic acid) may be referred to as a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. Certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). When a first nucleic acid strand binds to a second nucleic acid strand made up of nucleotides that are complementary to those in the first strand, the two strands bind to form a double strand. In various embodiments, "nucleic acid sequencing data," "nucleic acid sequencing information," "nucleic acid sequence," "genomic sequence," "genetic sequence," or "fragment sequence," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA.

In various embodiments, a "polynucleotide", "nucleic acid", or "oligonucleotide" refers to a linear polymer of nucleosides (including deoxyribonucleosides, ribonucleosides, or analogs thereof) joined by internucleosidic linkages. Typically, a polynucleotide comprises at least three nucleosides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

The term "allele" as used herein refers to a genetic variation associated with a gene or a segment of DNA, i.e., one of two or more alternate forms of a DNA sequence occupying the same locus.

The term "locus" as used herein refers to a specific position on a chromosome or a nucleic acid molecule. Alleles of a locus are located at identical sites on homologous chromosomes.

As used herein, the term "short tandem repeat (STR) loci" refers to regions of the human genome which contains short, repetitive sequence elements. For example, for Human Identification (HID) applications repetitive sequence elements are 3 to 7 base pairs in length. The repeats at a given STR marker do not need to be perfect repeats.

As used herein, the terms "adapter" or "adapter and its complements" and their derivatives, refers to any linear oligonucleotide which can be ligated to a nucleic acid molecule of the disclosure. Optionally, the adapter includes a nucleic acid sequence that is not substantially complementary to the 3' end or the 5' end of at least one target sequences within the sample. In some embodiments, the adapter is substantially non-complementary to the 3' end or the 5' end of any target sequence present in the sample. In some embodiments, the adapter includes any single stranded or double-stranded linear oligonucleotide that is not substantially complementary to an amplified target sequence. In some embodiments, the adapter is substantially non-complementary to at least one, some or all of the nucleic acid molecules of the sample. In some embodiments, suitable adapter lengths are in the range of about 10-100 nucleotides, about 12-60 nucleotides and about 15-50 nucleotides in length. An adapter can include any combination of nucleotides and/or nucleic acids. In some aspects, the adapter can include one or more cleavable groups at one or more locations. In another aspect, the adapter can include a sequence that is substantially identical, or substantially complementary, to at least a portion of a primer, for example a universal primer. In some embodiments, the adapter can include a barcode or tag to assist with downstream cataloguing, identification or sequencing. In some embodiments, a single-stranded adapter can act as a substrate for amplification when ligated to an amplified target sequence, particularly in the presence of a polymerase and dNTPs under suitable temperature and pH.

As used herein, "DNA barcode" or "DNA tagging sequence" and its derivatives, refers to a unique short (e.g., 6-14 nucleotide) nucleic acid sequence within an adapter that can act as a 'key' to distinguish or separate a plurality of amplified target sequences in a sample. For the purposes of this disclosure, a DNA barcode or DNA tagging sequence can be incorporated into the nucleotide sequence of an adapter.

In some embodiments, the disclosure provides for amplification of multiple target-specific sequences from a population of target nucleic acid molecules. In some embodiments, the method comprises hybridizing one or more target-specific primer pairs to the target sequence, extending a first primer of the primer pair, denaturing the extended first primer product from the population of nucleic acid molecules, hybridizing to the extended first primer product the second primer of the primer pair, extending the second primer to form a double stranded product, and digesting the target-specific primer pair away from the double stranded product to generate a plurality of amplified target sequences. In some embodiments, the digesting includes partial digesting of one or more of the target-specific primers from the amplified target sequence. In some embodiments, the amplified target sequences can be ligated to one or more adapters. In some embodiments, adapters can include one or more DNA barcodes or tagging sequences. In some embodiments, amplified target sequences once ligated to an adapter can undergo a nick translation reaction and/or further amplification to generate a library of adapter-ligated amplified target sequences.

In some embodiments, the methods of the disclosure include selectively amplifying target sequences in a sample containing a plurality of nucleic acid molecules and ligating the amplified target sequences to at least one adapter and/or barcode. Adapters and barcodes for use in molecular biology library preparation techniques are well known to those of skill in the art. The definitions of adapters and barcodes as used herein are consistent with the terms used in the art. For example, the use of barcodes allows for the detection and analysis of multiple samples, sources, tissues or populations of nucleic acid molecules per multiplex reaction. A barcoded and amplified target sequence contains a unique nucleic acid sequence, typically a short 6-15 nucleotide sequence, that identifies and distinguishes one amplified nucleic acid molecule from another amplified nucleic acid molecule, even when both nucleic acid molecules minus the barcode contain the same nucleic acid sequence. The use of adapters allows for the amplification of each amplified nucleic acid molecule in a uniformed manner and helps reduce strand bias. Adapters can include universal adapters or propriety adapters both of which can be used downstream to perform one or more distinct functions. For example, amplified target sequences prepared by the methods disclosed herein can be ligated to an adapter that may be used downstream as a platform for clonal amplification. The adapter can function as a template strand for subsequent amplification using a second set of primers and therefore allows universal amplification of the adapter-ligated amplified target sequence. In some embodiments, selective amplification of target nucleic acids to generate a pool of amplicons can further comprise ligating one or more barcodes and/or adapters to an amplified target sequence. The ability to incorporate barcodes enhances sample throughput and allows for analysis of multiple samples or sources of material concurrently.

In this application, "reaction confinement region" generally refers to any region in which a reaction may be confined and includes, for example, a "reaction chamber," a "well," and a "microwell" (each of which may be used interchangeably). A reaction confinement region may include a region in which a physical or chemical attribute of a solid substrate can permit the localization of a reaction of interest, and a discrete region of a surface of a substrate that can specifically bind an analyte of interest (such as a discrete region with oligonucleotides or antibodies covalently linked to such surface), for example. Reaction confinement regions may be hollow or have well-defined shapes and volumes, which may be manufactured into a substrate. These latter types of reaction confinement regions are referred to herein as microwells or reaction chambers, and may be fabricated using any suitable microfabrication techniques. Reaction confinement regions may also be substantially flat areas on a substrate without wells, for example.

A plurality of defined spaces or reaction confinement regions may be arranged in an array, and each defined space or reaction confinement regions may be in electrical communication with at least one sensor to allow detection or measurement of one or more detectable or measurable parameter or characteristics. This array is referred to herein as a sensor array. The sensors may convert changes in the presence, concentration, or amounts of reaction by-products (or changes in ionic character of reactants) into an output signal, which may be registered electronically, for example, as a change in a voltage level or a current level which, in turn, may be processed to extract information about a chemical reaction or desired association event, for example, a nucleotide incorporation event. The sensors may include at least one chemically sensitive field effect transistor ("chemFET") that can be configured to generate at least one output signal related to a property of a chemical reaction or target analyte of interest in proximity thereof. Such properties can include a concentration (or a change in concentration) of a reactant, product or by-product, or a value of a physical property (or a change in such value), such as an ion concentration. An initial measurement or interrogation of a pH for a defined space or reaction confinement regions, for example, may be represented as an electrical signal or a voltage, which may be digitalized (e.g., converted to a digital representation of the electrical signal or the voltage). Any of these measurements and representations may be considered raw data or a raw signal.

In various embodiments, the phrase "base space" refers to a representation of the sequence of nucleotides. The phrase "flow space" refers to a representation of the incorporation event or non-incorporation event for a particular nucleotide flow. For example, flow space can be a series of values representing a nucleotide incorporation event (such as a one, "1") or a non-incorporation event (such as a zero, "0") for that particular nucleotide flow. Nucleotide flows having a non-incorporation event can be referred to as empty flows, and nucleotide flows having a nucleotide incorporation event can be referred to as positive flows. It should be understood that zeros and ones are convenient representations of a non-incorporation event and a nucleotide incorporation event; however, any other symbol or designation could be used alternatively to represent and/or identify these events and non-events. In particular, when multiple nucleotides are incorporated at a given position, such as for a homopolymer stretch, the value can be proportional to the number of nucleotide incorporation events and thus the length of the homopolymer stretch.

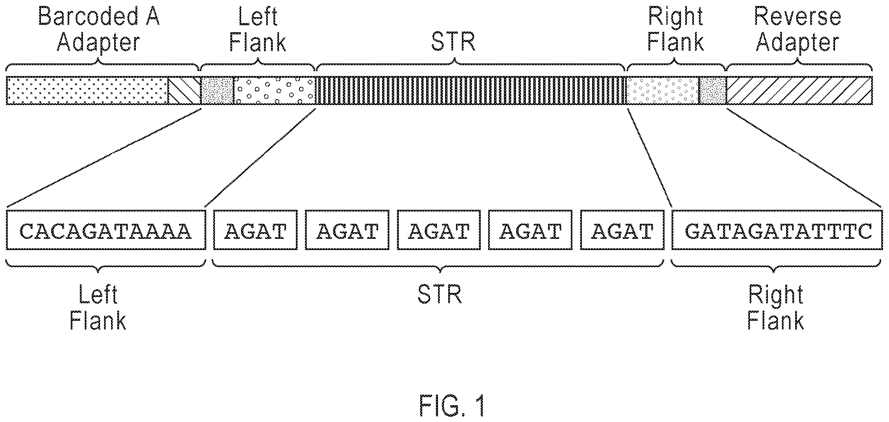

FIG. 1 illustrates an example of a sequence read having an STR region. The sequence read includes an STR region, or repeat region, surrounded by a left flank and a right flank. The STR region may include repeats of a short sequence of bases, or repeat sequence. In this example, the repeat sequence is [AGAT] and the number of repeats is 5, which can be represented as [AGAT]5. For Human Identification (HID) applications, the repeat sequence may be defined to have 3-7 bases. However, the embodiments described herein may be applied to longer repeat sequences having 2 bases or more than 7 bases. STR region is adjacent to a non-repetitive sequence of bases of the left flank and a non-repetitive sequence of bases of the right flank. The left flank is adjacent to the barcoded A adapter and the right flank is adjacent to the reverse adapter. In the example shown in FIG. 1, the A adapter and the reverse adapter bracket the target STR sequence such that template-dependent synthesis of the target sequence can be initiated from a forward primer hybridized to the A adapter sequence and/or from a reverse primer hybridized to the reverse adapter sequence.

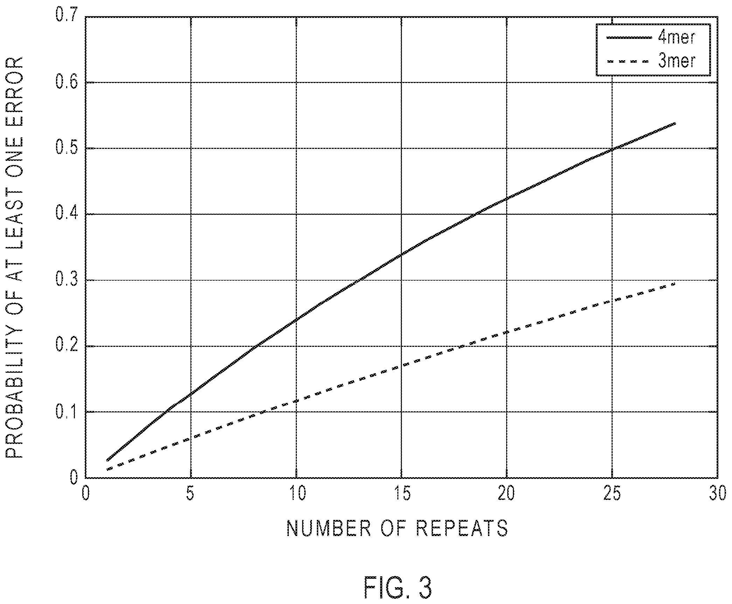

FIG. 3 is an example of error behavior when the repeat sequence of bases includes a 3-mer or 4-mer homopolymer. In this example, the 3-mers have an accuracy of 98.76% and the 4-mers have an accuracy of 97.3%. The plots show the probability of at least one error in the STR region increases as the number of repeats increases. The increase in probability of at least one error is greater for the 4-mer than for the 3-mers. The embodiments described herein correct insertion errors or deletion errors in homopolymers of the repeat sequences.

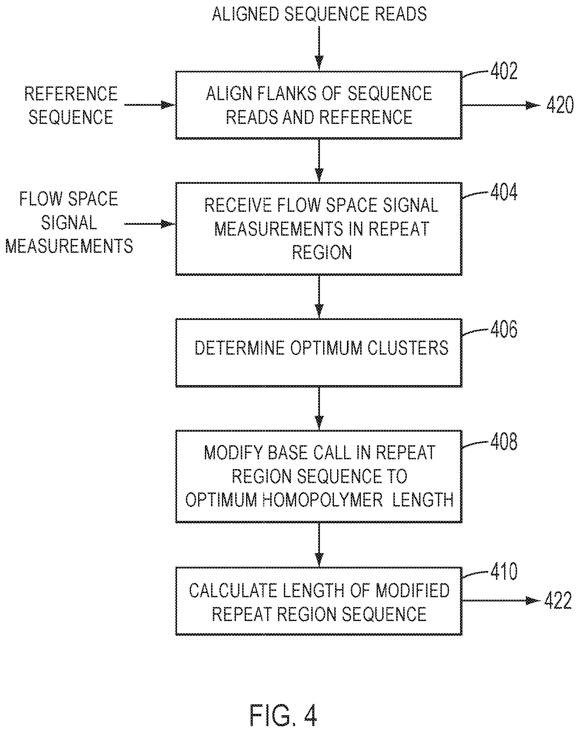

FIG. 4 illustrates a method of nucleic acid sequence analysis according to an exemplary embodiment. In the flank alignment step 402, a processor receives aligned sequence reads and a reference sequence. The aligned sequence reads can be retrieved from a file using a BAM file format, for example. The aligned sequence reads may correspond to a marker region of interest. The sequence reads include a sequence of bases of a left flank, a sequence of bases of a right flank and a repeat region of bases positioned between a rightmost base of the left flank and a leftmost base of the right flank. The repeat region includes an unknown number of repeats of a repeat sequence of bases. The processor aligns at least a portion of the sequence of bases of the left flank and at least a portion of the sequence of bases of the right flank with a left flank and right flank, respectively, of the reference sequence. The reference's left flank and right flank border the reference's repeat region corresponding to the marker region. The flank alignment step provides a set of repeat region sequences and adjacent left flank sequences and adjacent right flank sequences associated with the marker region. The processor may store the set of repeat region sequences and left and right flank sequences in a file 420 in memory. For example, the set of repeat region sequences and left and right flank sequences can be stored in a file 420 using the BAM file format.

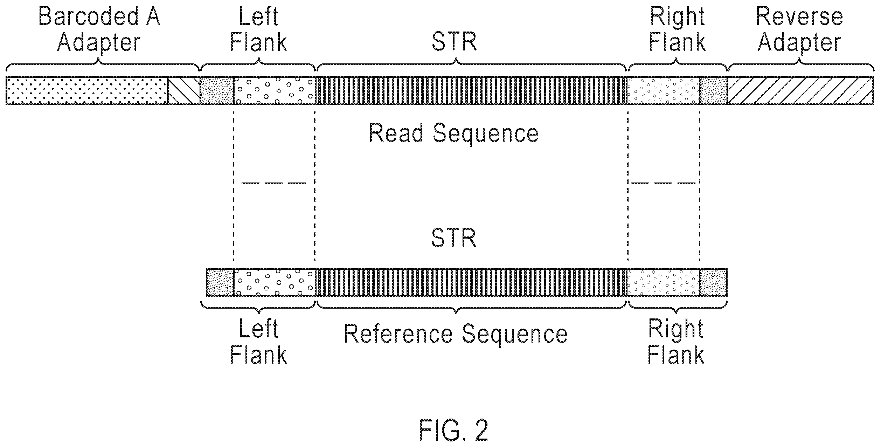

FIG. 2 illustrates an example of alignment of left and right flank regions of a sequence read with those of a reference. The alignment step 402 may map at least portions of the left and right flanks adjacent to the repeat region to the reference left and right flanks. FIG. 2 shows an example where a portion of the left flank is aligned with a portion of the reference left flank and a portion of the right flank is aligned with a portion of the reference right flank. The alignment step 402 may use a Smith-Waterman algorithm or other suitable mapping algorithm (see, e.g., Smith and Waterman, Journal of Molecular Biology 147(10:195-197 (1981)).

Returning to FIG. 4, in receiving step 404, the processor receives flow space signal measurements corresponding to the set of repeat region sequences. In some embodiments, each flow space signal measurement represents a signal amplitude or intensity measured in response to an incorporation or non-incorporation of a flowed nucleotide by sample nucleic acids in microwells of a sensor array. For an incorporation event, the signal amplitudes depend on the number of bases incorporated at one flow. For homopolymers, the signal amplitudes increase with increasing homopolymer length.

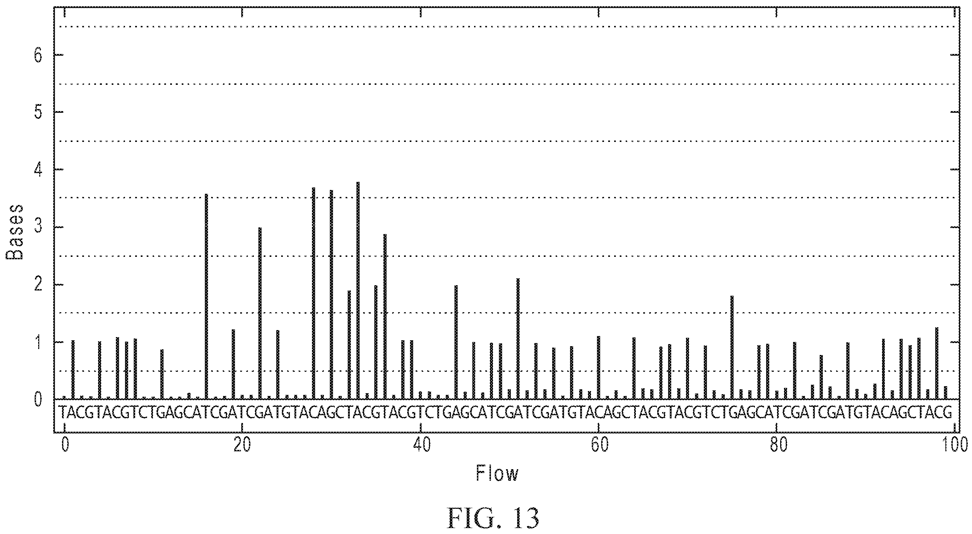

Figure 13 shows an exemplary representation of flow space signal measurements from which base calls may be made. In this example, the x-axis shows the flow number and nucleotide that was flowed in a flow sequence. The bars in the graph show the amplitudes of the flow space signal measurements for each flow from a particular location of a microwell in the sensor array. The numerals on the y-axis show the corresponding number of nucleotide incorporations that may be estimated by rounding to the nearest integer, for example. The number of nucleotide incorporations indicates a homopolymer length. The flow space signal measurements may be raw acquisition data or data having been processed, such as, e.g., by scaling, background filtering, normalization, correction for signal decay, and/or correction for phase errors or effects, etc. The base calls may be made by analyzing any suitable signal characteristics (e.g., signal amplitude or intensity). The structure and/or design of sensor array, signal processing and base calling for use with the present teachings may include one or more features described in U.S. Pat. Appl. Publ. No. 2012/0173159, published July 5, 2012,

For example, the nucleotide flow order is:

ACTGACTGA

and the respective signals generated by a well after each nucleotide flow are:

0.1, 0.3, 0.2, 1.4, 0.3, 1.2, 0.8, 1.5, 0.7

Based on the nucleotide flow sequence, a putative nucleic acid sequence is generated using the signals rounded to the nearest integer (as either a nucleotide incorporation event occurred or did not occur, but not partially). Thus, the above nucleotide flow order and signals establish a putative nucleic acid sequence as follows:

| FLOW | SEQUENCE | BASE SEQUENCE |
|---|---|---|
| A | 0.1 | |
| C | 0.3 | |
| T | 0.2 | |
| G | 1.4 | → G |
| A | 0.3 | |
| C | 1.2 | → C |
| T | 0.8 | → T |
| G | 1.5 | → G |

(continued)

| FLOW | SEQUENCE | BASE SEQUENCE |
|------|----------|---------------|
| A | 0.7 | → A |

Once the base sequence for the sequence read is determined, the sequence read may be aligned to a reference sequence to form aligned sequence reads. Methods for forming aligned sequence reads for use with the present teachings may include one or more features described in U.S. Pat. Appl. Publ. No. 2012/0197623, published August 2, 2012. The aligned sequence reads are provided to the flank alignment step 402, for example, in a BAM file.

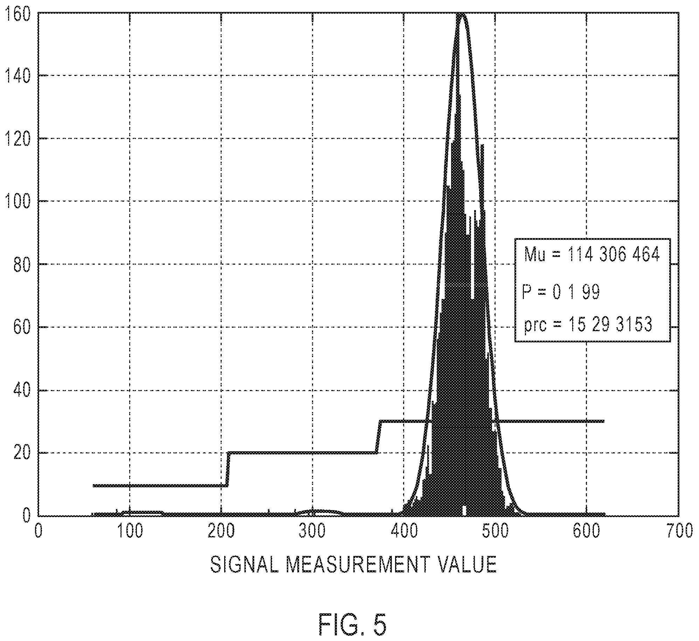

FIG. 5 shows an example of a flow space histogram of the flow space signal measurements for a single flow and where the repeat sequence includes 2-mer homopolymer. The histogram shows a large population centered at a mean value of 464 for the flow space signal measurements corresponding to the 2-mer. Two very small clusters are centered at mean values of 306 and 114. In the graph, the "prc" values indicate the number of flow space signal measurements assigned to each cluster, the "P" values indicate the percentage of total flow space signal measurements in each cluster, and "Mu" indicates the mean values of the clusters. The horizontal lines indicate the cluster range.

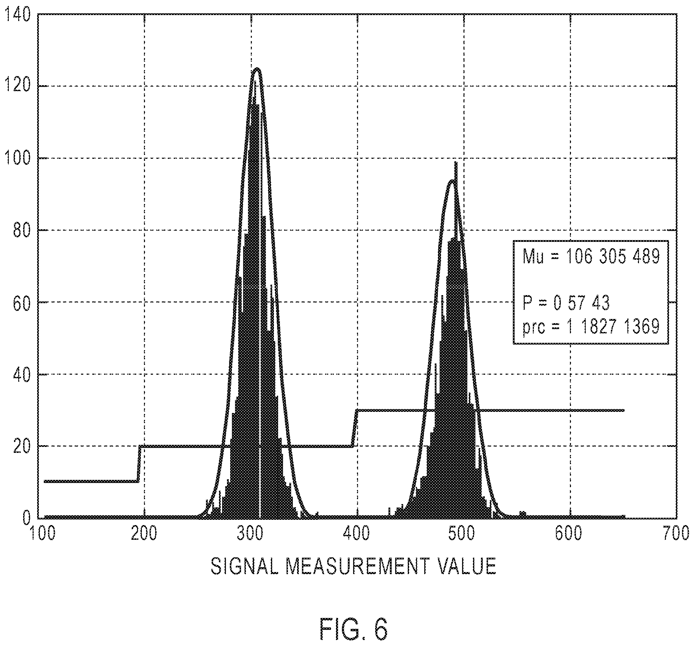

FIG. 6 shows an example of a flow space histogram of the flow space signal measurements for one flow, where the repeated sequence includes 2-mer homopolymer and two alleles are present. This histogram shows two large populations centered at mean values of 305 and 489. The repeat sequence [AATG] includes 2-mer homopolymer. The two populations in the histogram indicate the presence of two alleles. The population centered at 489 represents flow space signal measurements of the 2-mer AA in one allele. The population centered at 305 represents flow space signal measurements of a 1-mer A in the second allele.

Returning to FIG. 4, in the clustering step 406, the processor applies a clustering algorithm to determine optimum clusters for the flow space signal measurements. In some embodiments, the clustering step 406 may include generating a mixture model of probability density functions and corresponding membership parameters, or proportion parameters, wherein each of the probability density functions is associated with a cluster. The clustering step 406 may include maximizing a probability of the mixture model for the set of flow space signal measurements with respect to the corresponding membership parameters to form optimum clusters. An optimum cluster may be associated with a homopolymer length.

In some embodiments, the mixture model may comprise a Gaussian mixture model where the probability density functions are modeled as Gaussian. The clustering algorithm may apply an expectation maximization (EM) algorithm to determine the optimum clusters (see, for example, Dempster, A.P.; Laird, N.M.; Rubin, D.B. (1977). "Maximum Likelihood from Incomplete Data via the EM Algorithm". Journal of the Royal Statistical Society, Series B. 39 (1): 1-38. JSTOR 2984875. MR 0501537).

For a given flow, it may be assumed that the flow space signal measurements are modeled as a mixture Gaussian probability density functions. Each probability density function (pdf) has parameters of mean and standard deviation. The EM algorithm includes iteratively repeated steps: (1) identify membership of each flow space signal measurement in one of the Gaussian pdf's, (2) calculate the parameters of each Gaussian pdf, such as the mean and standard deviation, based on its member flow space signal measurements, and (3) calculate the log likelihood of the mixture model using the current values of the parameters for the Gaussian pdfs and the membership parameters. The steps may be iteratively repeated until a measure of convergence is achieved. Alternatively, the steps may be iteratively repeated for a fixed number of iterations.

The EM algorithm for a mixture of Gaussian pdf's can be written as follows:

$$P(X) = \prod_{i=1}^{n} p(x_i) = \prod_{i=1}^{n} \sum_{k=1}^{K} \pi_k F(x_i \mid \Theta_k) \qquad (1)$$

where, $P(X)$ = pdf for a set of flow space signal measurements under consideration for a single flow, $x_i$ = $i$th individual flow space signal measurement, $n$ = number of flow space signal measurements under consideration for a single flow, $p(x_i)$ = probability of $x_i$ using the mixture model, $k$ = cluster index, $F(x_i \mid \Theta_k)$ = pdf for $k$th cluster, $\Theta_k$ = vector of parameters for $k$th cluster (including mean and standard deviation), $\pi_k$ = membership parameter (or proportion parameter) for a proportion of flow space signal measurements in $k$th cluster where,

$$\sum_{k=1}^{K} \pi_k = 1$$

(2)

The goal of the EM algorithm is to find $\pi_k$ and $\Theta_k$ for $k = 1$ to $K$ such that $P(X)$ is maximized, or $ln(P(X)) = L(\Theta)$, or log likelihood, is maximized:

$$L(\Theta) = \sum_{i=1}^{n} \ln\left\{ \sum_{k=1}^{K} \pi_k F(x_i \mid \Theta_k) \right\}$$

$$(3)$$

For the optimum clusters, a $k^{th}$ cluster may correspond to a $k^{th}$ homopolymer length. For example, in FIG. 6, the cluster centered at 489 corresponds to a 2-mer and the cluster centered at 305 corresponds to a 1-mer.

In some embodiments, the mixture model may comprise a mixture of t-distributions where the probability density functions are modeled as t-distributions. The EM algorithm is then applied as above where the t-distribution is substituted for the Gaussian pdf. The EM algorithm may determine the optimum clusters using membership parameters and parameters of the t-distributions.

In some embodiments, an initial homopolymer length of a majority of initial base calls can be assigned to the largest cluster at each flow. In some embodiments, an initial expected value of the flow space signal measurements for a given homopolymer length can be used to initially associate a cluster with a particular homopolymer length. As the EM algorithm converges, the mean of the optimum cluster changes from the initial expected value. Example initial expected values are given in Table 1:

TABLE 1.

| HOMOPOLYMER LENGTH | SIGNAL MEASUREMENT EXPECTED VALUE |
|---|---|
| 0 | 0 |
| 1 | 255 |
| 2 | 510 |
| 3 | 750 |
| 4 | 900 |

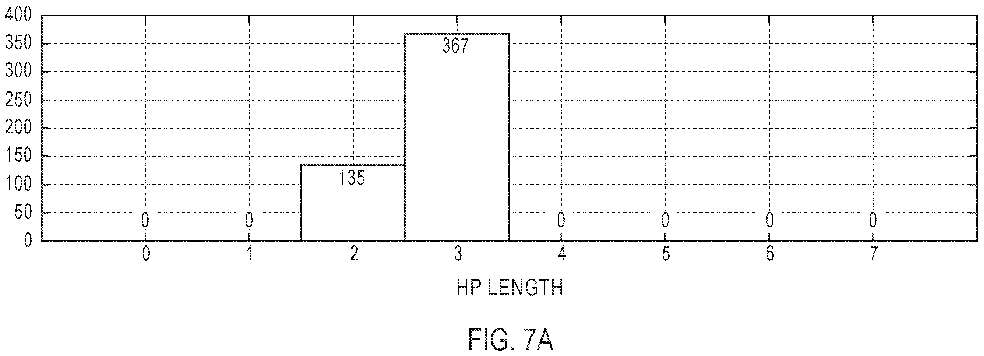

In some embodiments, the mixture model may be initialized with clusters corresponding to the homopolymer lengths observed in the base space histogram of previous flows. Initial means for the initial clusters may be set to expected values of flow space signal measurements for the observed homopolymer lengths in base space. For example, for 2-mer and 3-mer observations as in the base space histogram of FIG. 7A, two initial clusters can be defined for the Gaussian mixture model, each with a mean set to an expected value of the flow space signal measurement for 2-mers and 3-mers, respectively. In some embodiments, the mixture model may be initialized with a fixed number of clusters with means at locations within the range of flow space signal measurement values in the flow space histogram.

In some embodiments, the EM algorithm can be iteratively repeated for a fixed number of iterations, such as 9-10 iterations. The fixed number of iterations may also be set to fewer than 9 or greater than 10 iterations. In some embodiments, the EM algorithm can be iteratively repeated until a convergence threshold is satisfied. For example, the change in the log likelihood value, $L(\Theta)$, can be calculated from iteration to iteration and compared to a convergence threshold. The iterations can be stopped when the change in the log likelihood value, $L(\Theta)$, is less than the convergence threshold. For example, the convergence threshold can be based on a percent change in the log likelihood value, $L(\Theta)$, at a particular iteration compared to a previous iteration. The convergence threshold based on a percent change in the log likelihood can be set to a value in a range of 1-2%. In some embodiments, convergence threshold may be based the mean values of the clusters from iteration to iteration. For example, calculating an absolute value of a difference between the mean values for the cluster at a particular iteration compared to a previous iteration and comparing the percent change in mean value to a convergence threshold can determine convergence. The convergence threshold based on a percent change in mean value can be set to a value in a range of 0.5-1%.

In some embodiments, clustering algorithms such as K-means or Fuzzy-C-mean (FCM) classification may be applied in step 406 to determine optimum clusters. These clustering algorithms do not assume a Gaussian mixture model. These clustering algorithms classify flow space signal measurements into clusters with similar values at a specific flow. For example, FCM classification uses the data to estimate the centers of each cluster, then uses the new centers of the clusters to reassign the data to the clusters.

Returning to FIG. 4, in the base call modification step 408, the processor may modify an initially assigned base call at the position in the repeat region sequence to the homopolymer length associated with the optimum cluster for the flow space signal measurements for the given flow. Modifying the base call can correct an insertion error or a deletion error to produce a corrected repeat region sequence and length. In some situations, the initial base calls for a repeat region sequence may have insertion or deletion error in the repeated sequence, causing noisy alleles and error in STR length determinations. Modifying the base call to the homopolymer length associated with the optimum cluster can reduce the noisy alleles and improve the accuracy of STR length determinations. In step 410, the processor may calculate the length, or number of repeats, of the corrected repeat region sequence to produce corrected STR length values. The corrected STR length values may be stored in a file 422 in memory. The corrected number of repeats and corrected repeat region sequence may be used to confirm the presence of different alleles in a sample. For example, the corrected repeat region sequence of one allele may have an insertion or deletion in the repeat region, while the corrected repeat region sequence of another allele may not.

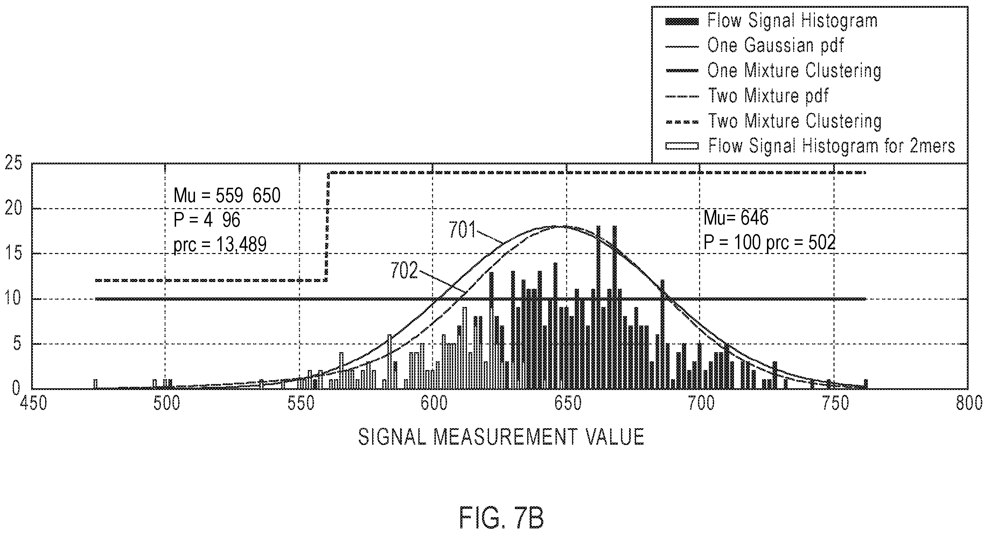

FIG. 7A shows an example of a base space histogram of base calls versus homopolymer length, where the base calls were made for signal measurements at a single flow. The histogram shows that 135 2-mers (27%) and 367 3-mers (73%) were called for a particular location in the repeat sequence. The base space histogram alone could indicate the presence of two alleles in the sample. FIG. 7B shows an example of the flow space histogram for the same signal measurements corresponding the base space histogram of FIG. 7A. The light bars indicate those signal measurements corresponding to the 2-mer homopolymer base calls in FIG. 7A. The superimposed curve 701 illustrates a Gaussian pdf modeling of a one mixture cluster with mean of 646. The superimposed curve 702 illustrates a Gaussian pdf modeling the larger cluster of a two mixture clustering model. The two mixture clustering model resulted in a large cluster having a mean of 650 and 96 percent of the signal measurements and a small cluster having a mean of 559 and 4 percent of the signal measurements. Comparing results, base space histogram of FIG. 7A indicates 73% of the base calls are 3-mers while the flow space histogram of FIG. 7B indicates that at least 96% of the signal measurements correspond to a 3-mer.

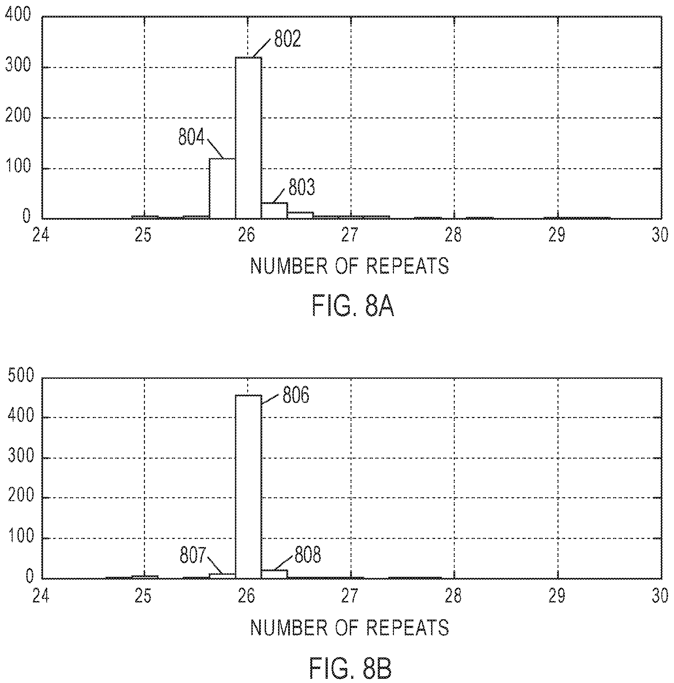

FIG. 8A shows an example of a histogram of the number of repeats, where the repeat region sequences resulted from base calling. Most of the repeat region sequences have 26 repeats, shown by bar 802. Bar 803 shows the number of repeat region sequences having 1 base more than 26 complete repeats and bar 804 shows the number of repeat region sequences having 1 base less than 26 complete repeats. FIG. 8B shows an example of a histogram of the number of repeats, where the repeat region sequences include base correction, in accordance with an exemplary embodiment. The results show a greater number of repeat region sequences having 26 repeats (bar 806), while the adjacent categories for one less base (bar 807) and one more base (bar 808) are reduced. FIGS. 7A, 7B, 8A and 8B present results for the marker D18S51, having repeat structure [AGAA].

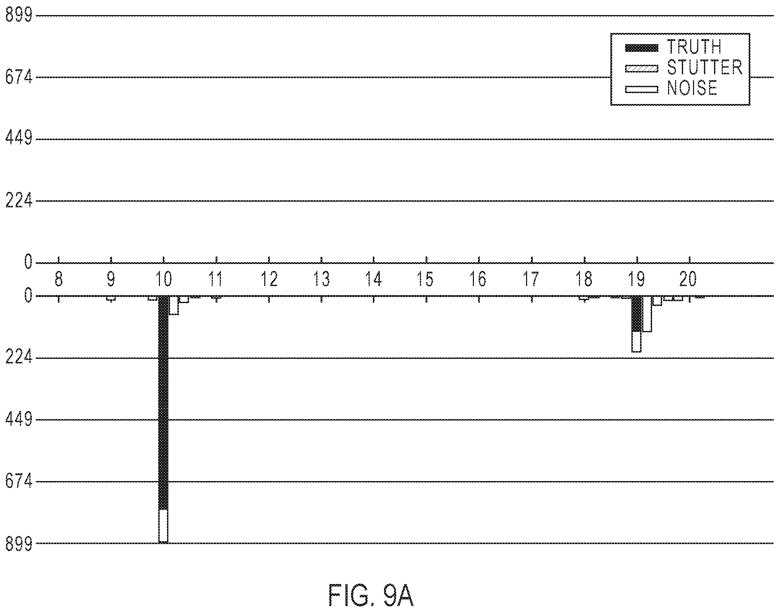

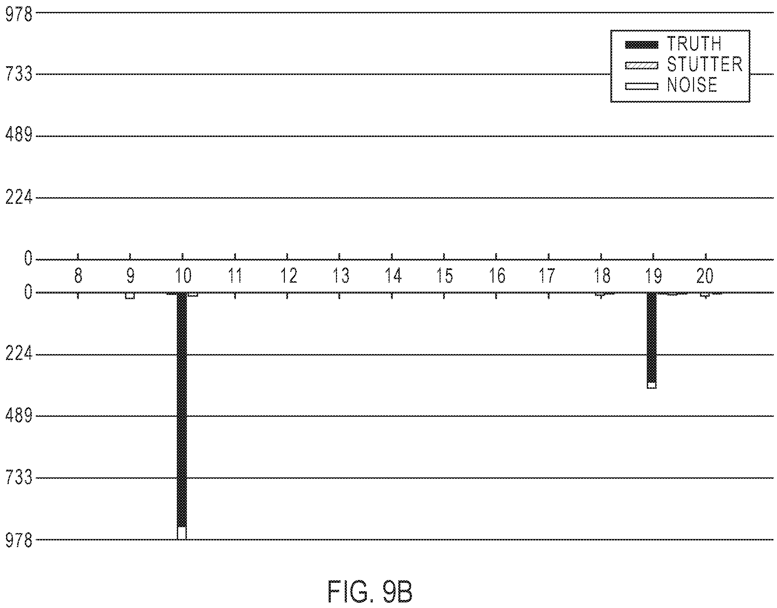

FIG. 9A shows an example of a histogram of the number of repeats, where the repeat region sequences resulted from base calling. FIG. 9B shows an example of a histogram of the number of repeats where the repeat region sequences include base correction, in accordance with an exemplary embodiment. The histograms are displayed for reverse sequencing. The histogram after base correction in FIG. 9B shows greater numbers of repeat region sequences at repeat lengths of 10 and 19 and a reduction in noise artifacts compared to the histogram of FIG. 9A. The FIGS. 9A and 9B give results for the marker PentaD having repeat structure [AAAGA].

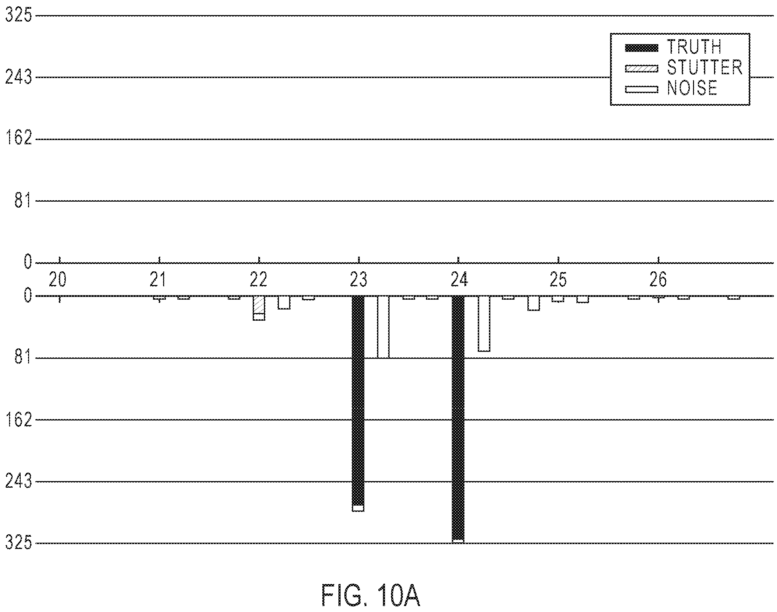

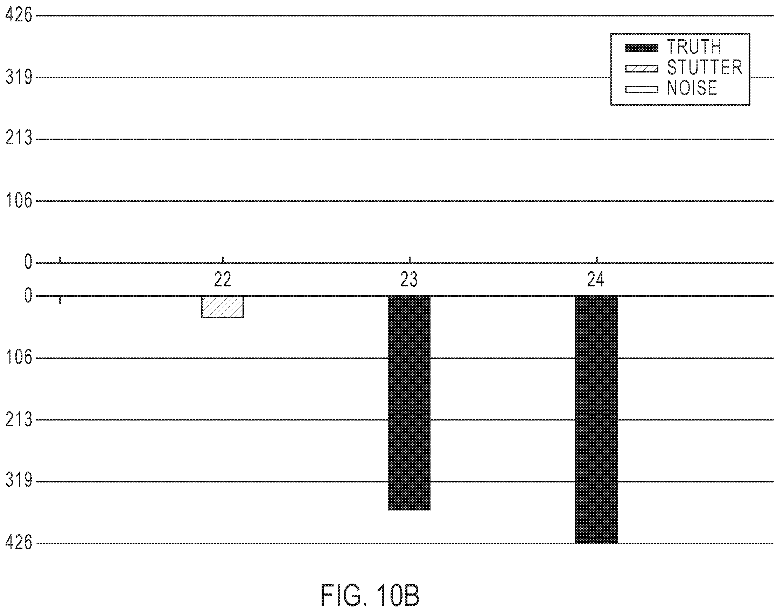

FIG. 10A shows an example of a histogram of the number of repeats, where the repeat region sequences resulted from base calling. FIG. 10B shows an example of a histogram of the number of repeats where the repeat region sequences include base correction, in accordance with an exemplary embodiment. The histograms are shown for reverse sequencing. The histogram after base correction in FIG. 10B shows greater numbers of repeat region sequences at repeat lengths of 23 and 24 and a reduction in the noise artifacts compared to the histogram of FIG. 10A. The FIGS. 10A and 10B give results for the marker FGA having a compound repeat structure [CTTT/TTCC].

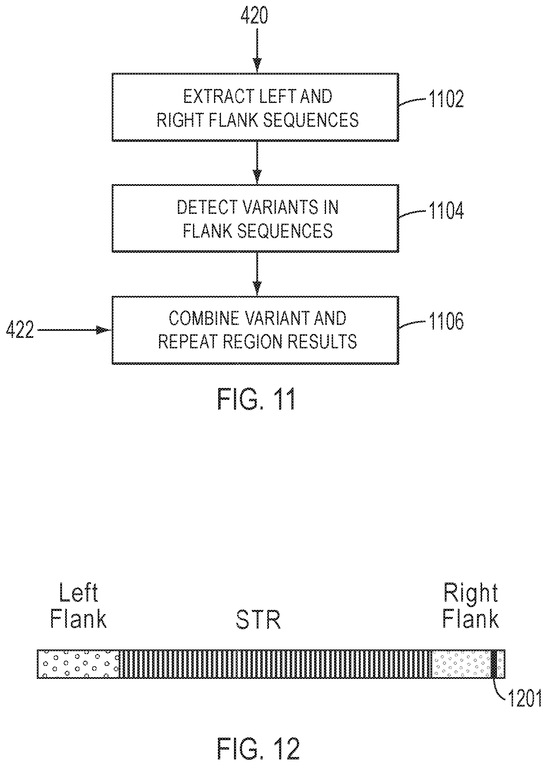

FIG. 11 is a block diagram of a method of nucleic acid sequence analysis to detect variants in flanks of repeat region sequences, according to an exemplary embodiment. In step 1102, a processor extracts the left flank sequences and the right flank sequences from the set of repeat region sequences and adjacent left flank sequences and adjacent right flank sequences corresponding to a given marker, provided by step 402 in file 420. The processor realigns the extracted left flank sequences and the extracted right flank sequences to the reference left flank and reference right flank, respectively, to produce aligned left flank sequences and aligned right flank sequences. The aligned left and right flank sequences may be stored in a file using the BAM file format. In the variant detection step 1104, a applies a variant caller to determine variations, including SNPs, insertions and deletions (indels), that may be present in the aligned left flank sequences and aligned right flank sequences. In some embodiments, the variant detection methods for use with the present teachings may include one or more features described in U.S. Pat. Appl. Publ. No. 2013/0345066, published December 26, 2013, U.S. Pat. Appl. Publ. published February 20, 2014, In some embodiments, other variant detection methods may be used. In step 1106, the processor combines variant detection information for the left flanks and the right flanks with the corrected STR length values provided by step 410 in file 422. The corrected STR length values variant detection information correspond to the same location in the sensor array. The combined results may be presented in a report to a user and/or stored in a file.

FIG. 12 illustrates an example of a variant 1201 in a right flank of an STR region. Table 2 gives an example of the combined variant and repeat region results.

TABLE 2.

| Allele | Coverage | Sequence | Long Sequence | Ref | SNP/indel Location | Quality Score |
|---|---|---|---|---|---|---|
| 8 | 293 | [GATA]8 | D16S539[CE8]-chr16-hg19 86386308-86386351 [GATA]8 | | | |
| | | | | | | |
| 9 | 3704 | [GATA]9 | D16S539[CE9]-chr16-hg19 86386308-86386351 [GATA]9 | | | |
| 9 | 3148 | [GATA]9 | D16S539[CE9]-chr16-hg19 86386308-86386351 [GATA]9 | | | |
| 9 | 520 | [GATA]9 | D16S539[CE9]-chr16-hg19 86386308-86386351 [GATA]9 86386367-C | A/C | Right Flank | 17.44 |
| | | | | | | |
| 10 | 4911 | [GATA]10 | D16S539[CE10]-chr16-hg19 86386308-86386351 [GATA]10 | | | |
| 10 | 4803 | [GATA]10 | D16S539[CE10]-chr16-hg19 86386308-86386351 [GATA]10 86386367-C | A/C | Right Flank | 17.2 |

In Table 2, alleles with the same STR length are grouped together. So the "Allele" column lists the STR lengths of 9, 10 and 11. The coverage is the number of sequence reads that support a specific allele. The "Sequence" column gives the repeat sequence and the number of repeats. The "Long Sequence" column gives the locus name and capillary electrophoresis allele name, chromosome and human genome assembly version, STR repeat region coordinates (start and end) for the reference allele, description of STR motif, and location of flanking region variant. In this example, 86386367-C identifies the variant base "C" at the location. The "Ref" column indicates the variant base C replaced the reference base A. The location column indicates the variant is located in the right flank of the STR region. The "Quality Score" column gives the quality score for the variant provided by the variant caller.

Table 3 gives a description of the marker regions used for the results described herein.

TABLE 3.

| Locus | Repeat Type | Repeat Structure | Core Loci | FIG. or TABLE |
|---|---|---|---|---|
| D16S539 | Simple | GATA | CODIS | TABLE 2 |
| D18S51 | Simple | AGAA | CODIS | FIGS. 7A, 7B, 8A, 8B |
| FGA | Compound | CTTT/TTCC | CODIS | FIGS. 10A, 10B |
| PentaD | Simple | AAAGA | STRidER | FIGS. 9A, 9B |

The Combined DNA Index System (CODIS) DNA database operated by the Federal Bureau of Investigation stores the DNA profile information of selected individuals. The profile includes 13 STR markers (13 loci with STR repeats), two additional allelic markers and AMEL, a sex determination allele. STRidER (STRs for identity ENFSI Reference database) is the European Network of Forensic Science Institutes DNA Working Group (ENFSI DNA WG) STR Population Database (https://strider.online/).

According to an exemplary embodiment, there is provided a method of nucleic acid sequence analysis, including: (1) receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases; (2) for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region; (3) receiving a plurality of flow space signal measurements corresponding to the set of repeat region sequences; (4) determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and (5) modifying a base call at the position in the repeat region sequence to the homopolymer length associated with the optimum cluster for the flow space signal measurements for the given flow to produce a corrected repeat region sequence, thereby correcting an insertion error or a deletion error. The method may further comprise calculating a number of repeats for the corrected repeat region sequence. The step of determining one or more optimum clusters may further comprise generating a mixture model of probability density functions, wherein each of the probability density functions is associated with a cluster of flow space signal measurements and a membership parameter. The probability density functions may comprise Gaussian probability density functions. The step of determining one or more optimum clusters may further comprise maximizing a probability of the mixture model for the set of flow space signal measurements for the given flow with respect to the membership parameters to form the optimum clusters. The step of maximizing a probability of the mixture model may further comprise applying an expectation maximization to a Gaussian mixture model. The step of calculating a number of repeats may further comprise applying a variant caller to the first sequence of bases of the left flank and the second sequence of bases of the right flank corresponding to the corrected repeat region sequence to determine a variant type and a variant location. The step of applying a variant caller may further comprise combining results for the number of repeats for the corrected repeat region sequence and the variant type and the variant location for the left flank and the right flank corresponding to the corrected repeat region sequence.

According to an exemplary embodiment, there is provided a system for nucleic acid sequence analysis, including a processor configured to perform the steps including: (1) receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases; (2) for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region; (3) receiving a plurality of flow space signal measurements corresponding to the set of repeat region sequences; (4) determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and (5) modifying a base call at the position in the repeat region sequence to the homopolymer length associated with the optimum cluster for the flow space signal measurements for the given flow to produce a corrected repeat region sequence, thereby correcting an insertion error or a deletion error. The method may further comprise calculating a number of repeats for the corrected repeat region sequence. The step of determining one or more optimum clusters may further comprise generating a mixture model of probability density functions, wherein each of the probability density functions is associated with a cluster of flow space signal measurements and a membership parameter. The probability density functions may comprise Gaussian probability density functions. The step of determining one or more optimum clusters may further comprise maximizing a probability of the mixture model for the set of flow space signal measurements for the given flow with respect to the membership parameters to form the optimum clusters. The step of maximizing a probability of the mixture model may further comprise applying an expectation maximization to a Gaussian mixture model. The step of calculating a number of repeats may further comprise applying a variant caller to the first sequence of bases of the left flank and the second sequence of bases of the right flank corresponding to the corrected repeat region sequence to determine a variant type and a variant location. The step of applying a variant caller may further comprise combining results for the number of repeats for the corrected repeat region sequence and the variant type and the variant location for the left flank and the right flank corresponding to the corrected repeat region sequence.

According to an exemplary embodiment, there is provided a non-transitory machine-readable storage medium comprising instructions which, when executed by a processor, cause the processor to perform a method for nucleic acid sequence analysis, including: (1) receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases; (2) for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region; (3) receiving a plurality of flow space signal measurements corresponding to the set of repeat region sequences; (4) determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and (5) modifying a base call at the position in the repeat region sequence to the homopolymer length associated with the optimum cluster for the flow space signal measurements for the given flow to produce a corrected repeat region sequence, thereby correcting an insertion error or a deletion error. The method may further comprise calculating a number of repeats for the corrected repeat region sequence. The step of determining one or more optimum clusters may further comprise generating a mixture model of probability density functions, wherein each of the probability density functions is associated with a cluster of flow space signal measurements and a membership parameter. The probability density functions may comprise Gaussian probability density functions. The step of determining one or more optimum clusters may further comprise maximizing a probability of the mixture model for the set of flow space signal measurements for the given flow with respect to the membership parameters to form the optimum clusters. The step of maximizing a probability of the mixture model may further comprise applying an expectation maximization to a Gaussian mixture model. The step of calculating a number of repeats may further comprise applying a variant caller to the first sequence of bases of the left flank and the second sequence of bases of the right flank corresponding to the corrected repeat region sequence to determine a variant type and a variant location. The step of applying a variant caller may further comprise combining results for the number of repeats for the corrected repeat region sequence and the variant type and the variant location for the left flank and the right flank corresponding to the corrected repeat region sequence.

Nucleic acid sequence data can be generated using various techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

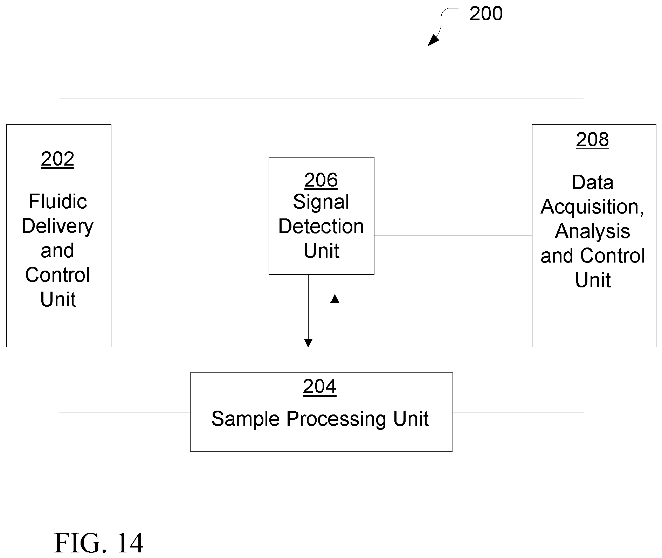

Various embodiments of nucleic acid sequencing platforms, such as a nucleic acid sequencer, can include components as displayed in the block diagram of FIG. 14. According to various embodiments, sequencing instrument 200 can include a fluidic delivery and control unit 202, a sample processing unit 204, a signal detection unit 206, and a data acquisition, analysis and control unit 208. Various embodiments of instrumentation, reagents, libraries and methods used for next generation sequencing are described in U.S. Patent Application Publication No. 2009/0127589 and No. 2009/0026082. Various embodiments of instrument 200 can provide for automated sequencing that can be used to gather sequence information from a plurality of sequences in parallel, such as substantially simultaneously.

In various embodiments, the fluidics delivery and control unit 202 can include reagent delivery system. The reagent delivery system can include a reagent reservoir for the storage of various reagents. The reagents can include RNA-based primers, forward/reverse DNA primers, oligonucleotide mixtures for ligation sequencing, nucleotide mixtures for sequencing-by-synthesis, optional ECC oligonucleotide mixtures, buffers, wash reagents, blocking reagent, stripping reagents, and the like. Additionally, the reagent delivery system can include a pipetting system or a continuous flow system which connects the sample processing unit with the reagent reservoir.

In various embodiments, the sample processing unit 204 can include a sample chamber, such as flow cell, a substrate, a micro-array, a multi-well tray, or the like. The sample processing unit 204 can include multiple lanes, multiple channels, multiple wells, or other means of processing multiple sample sets substantially simultaneously. Additionally, the sample processing unit can include multiple sample chambers to enable processing of multiple runs simultaneously. In particular embodiments, the system can perform signal detection on one sample chamber while substantially simultaneously processing another sample chamber. Additionally, the sample processing unit can include an automation system for moving or manipulating the sample chamber.

In various embodiments, the signal detection unit 206 can include an imaging or detection sensor. For example, the imaging or detection sensor can include a CCD, a CMOS, an ion or chemical sensor, such as an ion sensitive layer overlying a CMOS or FET, a current or voltage detector, or the like. The signal detection unit 206 can include an excitation system to cause a probe, such as a fluorescent dye, to emit a signal. The excitation system can include an illumination source, such as arc lamp, a laser, a light emitting diode (LED), or the like. In particular embodiments, the signal detection unit 206 can include optics for the transmission of light from an illumination source to the sample or from the sample to the imaging or detection sensor. Alternatively, the signal detection unit 206 may provide for electronic or non-photon based methods for detection and consequently not include an illumination source. In various embodiments, electronic-based signal detection may occur when a detectable signal or species is produced during a sequencing reaction. For example, a signal can be produced by the interaction of a released byproduct or moiety, such as a released ion, such as a hydrogen ion, interacting with an ion or chemical sensitive layer. In other embodiments a detectable signal may arise as a result of an enzymatic cascade such as used in pyrosequencing (see, for example, U.S. Patent Application Publication No. 2009/0325145) where pyrophosphate is generated through base incorporation by a polymerase which further reacts with ATP sulfurylase to generate ATP in the presence of adenosine 5' phosphosulfate wherein the ATP generated may be consumed in a luciferase mediated reaction to generate a chemiluminescent signal. In another example, changes in an electrical current can be detected as a nucleic acid passes through a nanopore without the need for an illumination source.

In various embodiments, a data acquisition analysis and control unit 208 can monitor various system parameters. The system parameters can include temperature of various portions of instrument 200, such as sample processing unit or reagent reservoirs, volumes of various reagents, the status of various system subcomponents, such as a manipulator, a stepper motor, a pump, or the like, or any combination thereof.

It will be appreciated by one skilled in the art that various embodiments of instrument 200 can be used to practice variety of sequencing methods including ligation-based methods, sequencing by synthesis, single molecule methods, nanopore sequencing, and other sequencing techniques.

In various embodiments, the sequencing instrument 200 can determine the sequence of a nucleic acid, such as a polynucleotide or an oligonucleotide. The nucleic acid can include DNA or RNA, and can be single stranded, such as ssDNA and RNA, or double stranded, such as dsDNA or a RNA/cDNA pair. In various embodiments, the nucleic acid can include or be derived from a fragment library, a mate pair library, a ChIP fragment, or the like. In particular embodiments, the sequencing instrument 200 can obtain the sequence information from a single nucleic acid molecule or from a group of substantially identical nucleic acid molecules.

In various embodiments, sequencing instrument 200 can output nucleic acid sequencing read data in a variety of different output data file types/formats, including, but not limited to: *.fasta, *.csfasta, *seq.txt, *qseq.txt, *.fastq, *.sff, *prb.txt, *.sms, *srs and/or *.qv.

According to various exemplary embodiments, one or more features of any one or more of the above-discussed teachings and/or exemplary embodiments may be performed or implemented using appropriately configured and/or programmed hardware and/or software elements. Determining whether an embodiment is implemented using hardware and/or software elements may be based on any number of factors, such as desired computational rate, power levels, heat tolerances, processing cycle budget, input data rates, output data rates, memory resources, data bus speeds, etc., and other design or performance constraints.

Examples of hardware elements may include processors, microprocessors, input(s) and/or output(s) (I/O) device(s) (or peripherals) that are communicatively coupled via a local interface circuit, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth. The local interface may include, for example, one or more buses or other wired or wireless connections, controllers, buffers (caches), drivers, repeaters and receivers, etc., to allow appropriate communications between hardware components. A processor is a hardware device for executing software, particularly software stored in memory. The processor can be any custom made or commercially available processor, a central processing unit (CPU), an auxiliary processor among several processors associated with the computer, a semiconductor based microprocessor (e.g., in the form of a microchip or chip set), a macroprocessor, or generally any device for executing software instructions. A processor can also represent a distributed processing architecture. The I/O devices can include input devices, for example, a keyboard, a mouse, a scanner, a microphone, a touch screen, an interface for various medical devices and/or laboratory instruments, a bar code reader, a stylus, a laser reader, a radio-frequency device reader, etc. Furthermore, the I/O devices also can include output devices, for example, a printer, a bar code printer, a display, etc. Finally, the I/O devices further can include devices that communicate as both inputs and outputs, for example, a modulator/demodulator (modem; for accessing another device, system, or network), a radio frequency (RF) or other transceiver, a telephonic interface, a bridge, a router, etc.

Examples of software may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. A software in memory may include one or more separate programs, which may include ordered listings of executable instructions for implementing logical functions. The software in memory may include a system for identifying data streams in accordance with the present teachings and any suitable custom made or commercially available operating system (O/S), which may control the execution of other computer programs such as the system, and provides scheduling, input-output control, file and data management, memory management, communication control, etc.

According to various exemplary embodiments, one or more features of any one or more of the above-discussed teachings and/or exemplary embodiments may be performed or implemented using appropriately configured and/or programmed non-transitory machine-readable medium or article that may store an instruction or a set of instructions that, if executed by a machine, may cause the machine to perform a method and/or operations in accordance with the exemplary embodiments. Such a machine may include, for example, any suitable processing platform, computing platform, computing device, processing device, computing system, processing system, computer, processor, scientific or laboratory instrument, etc., and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or re-writeable media, digital or analog media, hard disk, floppy disk, read-only memory compact disc (CD-ROM), recordable compact disc (CD-R), rewriteable compact disc (CD-RW), optical disk, magnetic media, magneto-optical media, removable memory cards or disks, various types of Digital Versatile Disc (DVD), a tape, a cassette, etc., including any medium suitable for use in a computer. Memory can include any one or a combination of volatile memory elements (e.g., random access memory (RAM, such as DRAM, SRAM, SDRAM, etc.)) and nonvolatile memory elements (e.g., ROM, EPROM, EEROM, Flash memory, hard drive, tape, CDROM, etc.). Moreover, memory can incorporate electronic, magnetic, optical, and/or other types of storage media. Memory can have a distributed architecture where various components are situated remote from one another, but are still accessed by the processor. The instructions may include any suitable type of code, such as source code, compiled code, interpreted code, executable code, static code, dynamic code, encrypted code, etc., implemented using any suitable high-level, low-level, object-oriented, visual, compiled and/or interpreted programming language.

According to various exemplary embodiments, one or more features of any one or more of the above-discussed teachings and/or exemplary embodiments may be performed or implemented at least partly using a distributed, clustered, remote, or cloud computing resource.

According to various exemplary embodiments, one or more features of any one or more of the above-discussed teachings and/or exemplary embodiments may be performed or implemented using a source program, executable program (object code), script, or any other entity comprising a set of instructions to be performed. When a source program, the program can be translated via a compiler, assembler, interpreter, etc., which may or may not be included within the memory, so as to operate properly in connection with the O/S. The instructions may be written using (a) an object oriented programming language, which has classes of data and methods, or (b) a procedural programming language, which has routines, subroutines, and/or functions, which may include, for example, C, C++, R, Pascal, Basic, Fortran, Cobol, Perl, Java, and Ada.

According to various exemplary embodiments, one or more of the above-discussed exemplary embodiments may include transmitting, displaying, storing, printing or outputting to a user interface device, a computer readable storage medium, a local computer system or a remote computer system, information related to any information, signal, data, and/or intermediate or final results that may have been generated, accessed, or used by such exemplary embodiments. Such transmitted, displayed, stored, printed or outputted information can take the form of searchable and/or filterable lists of runs and reports, pictures, tables, charts, graphs, spreadsheets, correlations, sequences, and combinations thereof, for example.

**Claims**

1.    A method of nucleic acid sequence analysis, carried out on a processor, comprising:

receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases;
for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region;
receiving a plurality of flow space signal measurements corresponding to the set of repeat region sequences;
determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and
modifying a base call at the position in the repeat region sequence to the homopolymer length associated with the optimum cluster for the flow space signal measurements for the given flow to produce a corrected repeat region sequence, thereby correcting an insertion error or a deletion error.

2. The method of claim 1, further comprising calculating a number of repeats for the corrected repeat region sequence.

3. The method of claim 1, wherein determining one or more optimum clusters further comprises generating a mixture model of probability density functions, wherein each of the probability density functions is associated with a cluster of flow space signal measurements and a membership parameter.

4. The method of claim 3, wherein the probability density functions comprise Gaussian probability density functions.

5. The method of claim 3, wherein determining one or more optimum clusters further comprises maximizing a probability of the mixture model for the set of flow space signal measurements for the given flow with respect to the membership parameters to form the optimum clusters.

6. The method of claim 5, wherein maximizing a probability of the mixture model further comprises applying an expectation maximization to a Gaussian mixture model.

7. The method of claim 2, further comprising applying a variant caller to the first sequence of bases of the left flank and the second sequence of bases of the right flank corresponding to the corrected repeat region sequence to determine a variant type and a variant location, and optionally further comprising combining results for the number of repeats for the corrected repeat region sequence and the variant type and the variant location for the left flank and the right flank corresponding to the corrected repeat region sequence.

8. A system for nucleic acid sequence analysis, comprising a processor configured to perform the steps including:

receiving a plurality of nucleic acid sequence reads corresponding to a marker region, wherein each of the sequence reads includes a first sequence of bases of a left flank, a second sequence of bases of a right flank and a repeat region of bases positioned between a rightmost base of the left flank and a leftmost base of the right flank, wherein the repeat region includes a number of repeats of a repeated sequence of bases;
for each of the sequence reads, aligning at least a portion of the first sequence of bases of the left flank adjacent to the repeat region with a reference left flank and at least a portion of the second sequence of bases of the right flank adjacent to the repeat region with a reference right flank, wherein the reference left flank and the reference right flank border a reference repeat region of a reference nucleic acid sequence corresponding to the marker region to form a set of repeat region sequences and adjacent left flank sequences and adjacent right flank sequences associated with the marker region;
receiving a plurality of flow space signal measurements corresponding to the set of repeat region sequences;
determining one or more optimum clusters for a set of flow space signal measurements of the plurality of flow space signal measurements, wherein at least one of the optimum clusters is associated with a homopolymer length, wherein the set of flow space signal measurements corresponds to a given flow and to a position in the repeat region sequence; and
modifying a base call at the position in the repeat region sequence to the homopolymer length associated with the optimum cluster for the flow space signal measurements for the given flow to produce a corrected repeat region sequence, thereby correcting an insertion error or a deletion error.

9. The system of claim 8, wherein the processor is further configured to perform a step including calculating a number of repeats for the corrected repeat region sequence.

10. The system of claim 8, wherein determining one or more optimum clusters further comprises generating a mixture model of probability density functions, wherein each of the probability density functions is associated with a cluster of flow space signal measurements and a membership parameter.

11. The system of claim 10, wherein the probability density functions comprise Gaussian probability density functions.

12. The system of claim 10, wherein determining one or more optimum clusters further comprises maximizing a probability of the mixture model for the set of flow space signal measurements for the given flow with respect to the membership parameters to form the optimum clusters.

13. The system of claim 12, wherein maximizing a probability of the mixture model further comprises applying an expectation maximization to a Gaussian mixture model.

14. The system of claim 9, wherein the processor is further configured to perform a step including applying a variant caller to the first sequence of bases of the left flank and the second sequence of bases of the right flank corresponding to the corrected repeat region sequence to determine a variant type and a variant location, and optionally
to perform a step including combining results for the number of repeats for the corrected repeat region sequence and the variant type and the variant location for the left flank and the right flank corresponding to the corrected repeat region sequence.

15. A non-transitory machine-readable storage medium comprising instructions which, when executed by a processor, cause the processor to perform a method for nucleic acid sequence analysis according to any one of claims 1 to 7.

**Patentansprüche**

1. Verfahren zur Analyse der Nukleinsäuresequenz, durchgeführt auf einem Prozessor, Folgendes umfassend:

Empfangen einer Vielzahl von Nukleinsäuresequenz-Reads, die einer Markerregion entsprechen, wobei jedes der Sequenz-Reads eine erste Basensequenz einer linken Flanke, eine zweite Basensequenz einer rechten Flanke und eine Wiederholungsregion von Basen umfasst, die zwischen einer Basenposition ganz rechts der linken Flanke und einer Basenposition ganz links der rechten Flanke angeordnet ist, wobei die Wiederholungsregion eine Anzahl von Wiederholungen einer wiederholten Basensequenz umfasst;
für jeden der Sequenz-Reads mindestens einen Teil der ersten Basensequenz der linken Flanke, die an die Wiederholungsregion angrenzt, mit einer Referenz-Linksflanke und mindestens einen Teil der zweiten Basensequenz der rechten Flanke, die an die Wiederholungsregion angrenzt, mit einer Referenz-Rechtsflanke abzugleichen, wobei die Referenz-Linksflanke und die Referenz-Rechtsflanke an eine Referenz-Wiederholungsregion einer Referenz-Nukleinsäuresequenz angrenzen, die der Markerregion entspricht, um einen Satz von Wiederholungsregionssequenzen und angrenzenden Linksflanken-Sequenzen und angrenzenden Rechtsflanken-Sequenzen zu bilden, die mit der Markerregion assoziiert sind;
Empfangen einer Vielzahl von Strömungsraumsignalmessungen, die dem Satz von Wiederholungsregionsequenzen entsprechen;
Bestimmen eines oder mehrerer optimaler Cluster für einen Satz von Strömungsraumsignalmessungen aus der Vielzahl von Strömungsraumsignalmessungen, wobei mindestens einer der optimalen Cluster mit einer Homopolymerlänge assoziiert ist, wobei der Satz von Strömungsraumsignalmessungen einer gegebenen Strömung und einer Position in der Wiederholungsbereichssequenz entspricht; und
Modifizieren eines Basen-Calls an der Position in der Wiederholungsregionsequenz auf die Homopolymerlänge, die dem optimalen Cluster für die Strömungsraumsignalmessungen für die gegebene Strömung zugeordnet ist, um eine korrigierte Wiederholungsregionsequenz zu erzeugen, wodurch ein Insertionsfehler oder ein Deletionsfehler korrigiert wird.

2. Verfahren nach Anspruch 1, weiterhin umfassend das Berechnung einer Anzahl von Wiederholungen für die korrigierte Wiederholungsregionsequenz.

3. Verfahren nach Anspruch 1, wobei das Bestimmen eines oder mehrerer optimaler Cluster weiterhin das Erzeugen eines Mischungsmodells von Wahrscheinlichkeitsdichtefunktionen umfasst, wobei jede der Wahrscheinlichkeitsdichtefunktionen einem Cluster von Strömungsraumsignalmessungen und einem Zugehörigkeitsparameter zugeordnet ist.

4. Verfahren nach Anspruch 3, wobei die Wahrscheinlichkeitsdichtefunktionen Gaußsche Wahrscheinlichkeitsdichtefunktionen umfassen.

5. Verfahren nach Anspruch 3, wobei das Bestimmen eines oder mehrerer optimaler Cluster ferner das Maximieren einer Wahrscheinlichkeit des Mischungsmodells für den Satz von Strömungsraumsignalmessungen für die gegebene Strömung in Bezug auf die Mitgliedschaftsparameter umfasst, um die optimalen Cluster zu bilden.

6. Verfahren nach Anspruch 5, wobei das Maximieren einer Wahrscheinlichkeit des Mischungsmodells ferner das Anwenden einer Erwartungsmaximierung auf ein Gaußsches Mischungsmodell umfasst.

**7.** Verfahren nach Anspruch 2, weiterhin umfassend das Anwenden eines Varianten-Callers auf die erste Basensequenz der linken Flanke und die zweite Basensequenz der rechten Flanke, die der korrigierten Wiederholungsregionsequenz entsprechen, um einen Variantentyp und eine Variantenposition zu bestimmen, und optional weiter umfassend das Kombinieren von Ergebnissen für die Anzahl der Repeats für die korrigierte Wiederholungsregionsequenz und den Variantentyp und die Variantenposition für die linke Flanke und die rechte Flanke, die der korrigierten Wiederholungsregionsequenz entsprechen.

**8.** System zur Nukleinsäuresequenzanalyse, umfassend einen Prozessor, der zur Durchführung der folgenden Schritte konfiguriert ist:

Empfangen einer Vielzahl von Nukleinsäuresequenz-Reads, die einer Markerregion entsprechen, wobei jedes der Sequenz-Reads eine erste Basensequenz einer linken Flanke, eine zweite Basensequenz einer rechten Flanke und eine Wiederholungsregion von Basen umfasst, die zwischen einer Basenposition ganz rechts der linken Flanke und einer Basenposition ganz links der rechten Flanke angeordnet ist, wobei die Wiederholungsregion eine Anzahl von Wiederholungen einer wiederholten Basensequenz umfasst;
für jedes der Sequenz-Reads mindestens einen Teil der ersten Basensequenz der linken Flanke, die an die Wiederholungsregion angrenzt, mit einer Referenz-linken Flanke und mindestens einen Teil der zweiten Basensequenz der rechten Flanke, die an die Wiederholungsregion angrenzt, mit einer Referenz-rechten Flanke abzugleichen, wobei die Referenz-Linksflanke und die Referenz-Rechsflanke an eine Referenz-Wiederholungsregion einer Referenz-Nukleinsäuresequenz angrenzen, die der Markerregion entspricht, um einen Satz von Wiederholungsregionssequenzen und angrenzenden Linksflanken-Sequenzen und angrenzenden Rechtsflanken-Sequenzen zu bilden, die mit der Markerregion assoziiert sind;
Empfangen einer Vielzahl von Strömungsraumsignalmessungen, die dem Satz von Wiederholungsregionsequenzen entsprechen;
Bestimmen eines oder mehrerer optimaler Cluster für einen Satz von Strömungsraumsignalmessungen aus der Vielzahl von Strömungsraumsignalmessungen, wobei mindestens einer der optimalen Cluster mit einer Homopolymerlänge assoziiert ist, wobei der Satz von Strömungsraumsignalmessungen einer gegebenen Strömung und einer Position in der Wiederholungsbereichssequenz entspricht; und
Modifizieren eines Basen-Calls an der Position in der Sequenz der Wiederholungsregion auf die Homopolymerlänge, die mit dem optimalen Cluster für das Strömungsraumsignal assoziiert ist.

Messungen für die angegebene Strömung, um eine korrigierte Wiederholungsregionensequenz zu erzeugen und dadurch einen Insertionsfehler oder einen Deletionsfehler zu korrigieren.

**9.** System nach Anspruch 8, wobei der Prozessor weiterhin dazu konfiguriert ist, einen Schritt durchzuführen, der das Berechnung einer Anzahl von Wiederholungen für die korrigierte Wiederholungsregionsequenz umfasst.

**10.** Verfahren nach Anspruch 8, wobei das Bestimmen eines oder mehrerer optimaler Cluster weiterhin das Erzeugen eines Mischungsmodells von Wahrscheinlichkeitsdichtefunktionen umfasst, wobei jede der Wahrscheinlichkeitsdichtefunktionen einem Cluster von Strömungsraumsignalmessungen und einem Zugehörigkeitsparameter zugeordnet ist.

**11.** Verfahren nach Anspruch 10, wobei die Wahrscheinlichkeitsdichtefunktionen Gaußsche Wahrscheinlichkeitsdichtefunktionen umfassen.

**12.** Verfahren nach Anspruch 10, wobei das Bestimmen eines oder mehrerer optimaler Cluster ferner das Maximieren einer Wahrscheinlichkeit des Mischungsmodells für den Satz von Strömungsraumsignalmessungen für die gegebene Strömung in Bezug auf die Mitgliedschaftsparameter umfasst, um die optimalen Cluster zu bilden.

**13.** Verfahren nach Anspruch 12, wobei das Maximieren einer Wahrscheinlichkeit des Mischungsmodells ferner das Anwenden einer Erwartungsmaximierung auf ein Gaußsches Mischungsmodell umfasst.

**14.** System nach Anspruch 9, wobei der Prozessor weiterhin konfiguriert ist, um einen Schritt durchzuführen, der das Anwenden eines Varianten-Callers auf die erste Sequenz von Basen der linken Flanke und die zweite Sequenz von Basen der rechten Flanke, die der korrigierten Wiederholungsregionsequenz entsprechen, umfasst, um einen Variantentyp und eine Variantenlokalisation zu bestimmen, sowie optional Durchführung eines Schritts, der das Kombinieren der Ergebnisse für die Anzahl der Wiederholungen für die korrigierte Wiederholungsregionsequenz und den Variantentyp und die Variantenstelle für die linke und die rechte Flanke, die der korrigierten Wiederholungsregionsequenz entsprechen, umfasst.

15. Nichtflüchtiges, maschinenlesbares Speichermedium, das Befehle enthält, die, wenn sie von einem Prozessor ausgeführt werden, den Prozessor veranlassen, ein Verfahren zur Nukleinsäuresequenzanalyse gemäß einem der Ansprüche 1 bis 7 durchzuführen.

**Revendications**

1. Procédé d'analyse de séquences d'acides nucléiques, réalisé par un processeur, comprenant:

   recevoir une série de lectures de séquence d'acides nucléiques correspondant à une région marqueur, dans laquelle chacune des lectures de séquence comprend une première séquence de bases d'un flanc gauche, une deuxième séquence de bases d'un flanc droit et une région de répétition de bases positionnée entre le niveau le plus à droite de la base du flanc gauche et du niveau le plus à gauche de la base du flanc droit, région où les répétitions comprennent un certain nombre de répétitions d'une séquence répétée de bases;
   pour chacune des lectures de séquence, aligner au moins une partie de la première séquence de bases du flanc gauche adjacent à la région de répétition avec un flanc gauche de référence et au moins une partie de la seconde séquence de bases du flanc droit adjacent à la région de répétition avec un flanc droit de référence, le flanc gauche de référence et le flanc droit de référence bordant une région de répétition de référence d'une séquence d'acide nucléique de référence correspondant à la région marqueur pour former un ensemble de séquences de région de répétition et de séquences de flanc gauche adjacentes et de séquences de flanc droit adjacentes associées à la région marqueur;
   recevoir une série de mesures de signaux d'espace de flux correspondant à l'ensemble de séquences de régions de répétition;
   déterminer un ou plusieurs groupes optimaux pour un ensemble de mesures de signaux d'espace de flux parmi la pluralité de mesures de signaux d'espace de flux, dans lequel au moins un des groupes optimaux est associé à une longueur d'homopolymère, dans lequel l'ensemble de mesures de signaux d'espace de flux correspond à un flux donné et à une position dans la séquence de la région de répétition; et
   modifier une définition des bases à la position dans la séquence de la région de répétition en longueur d'homopolymère associée au groupe optimal pour les mesures de signaux d'espace de flux pour le flux donné afin de produire une séquence d'insertion de région de répétition corrigée, corrigeant ainsi une erreur d'insertion ou une erreur de suppression.

2. Le procédé de revendication 1, comprenant en outre le calcul d'un nombre de répétitions pour la séquence de région de répétition corrigée.

3. Le procédé de revendication 1, dans lequel la détermination d'un ou plusieurs groupes optimaux comprend en outre la génération d'un modèle de mélange de fonctions de densité de probabilité, dans lequel chacune des fonctions de densité de probabilité est associée à un groupe de mesures de signaux d'espace de flux et à un paramètre d'appartenance.

4. Le procédé de revendication 3, dans lequel les fonctions de densité de probabilité comprennent des fonctions de densité de probabilité Gaussiennes.

5. Le procédé de revendication 3, dans lequel la détermination d'un ou plusieurs groupes optimaux comprend en outre la maximisation d'une probabilité du modèle de mélange pour l'ensemble de mesures de signaux d'espace de flux pour le flux donné en ce qui concerne les paramètres d'appartenance pour former les groupes optimaux.

6. Le procédé de revendication 5, dans lequel la maximisation d'une probabilité du modèle de mélange comprend en outre l'application d'une maximisation d'espérance à un modèle de mélange gaussien.

7. Le procédé de revendication 2, comprenant en outre l'application de l'appel de variant à la première séquence de bases du flanc gauche et à la seconde séquence de bases du flanc droit correspondant à la séquence de région de répétition corrigée pour déterminer un type de variant et un emplacement de variant, et comprenant en outre facultativement la combinaison des résultats pour le nombre de répétitions pour la séquence de région de répétition corrigée et le type de variant et l'emplacement de variant pour le flanc gauche et le flanc droit correspondant à la séquence de région de répétition corrigée.

8. Système d'analyse de séquences d'acides nucléiques, comprenant un processeur configuré pour effectuer les étapes comprenant:

recevoir une série de lectures de séquence d'acides nucléiques correspondant à une région marqueur, dans laquelle chacune des lectures de séquence comprend une première séquence de bases d'un flanc gauche, une deuxième séquence de bases d'un flanc droit et une région de répétition de bases positionnée entre le niveau le plus à droite de la base du flanc gauche et du niveau le plus à gauche de la base du flanc droit, région où les répétitions comprennent un certain nombre de répétitions d'une séquence répétée de bases;

pour chacune des lectures de séquence, aligner au moins une partie de la première séquence de bases du flanc gauche adjacent à la région de répétition avec un flanc gauche de référence et au moins une partie de la seconde séquence de bases du flanc droit adjacent à la région de répétition avec un flanc droit de référence, le flanc gauche de référence et le flanc droit de référence bordant une région de répétition de référence d'une séquence d'acide nucléique de référence correspondant à la région marqueur pour former un ensemble de séquences de région de répétition et de séquences de flanc gauche adjacentes et de séquences de flanc droit adjacentes associées à la région marqueur;

recevoir une série de mesures de signaux d'espace de flux correspondant à l'ensemble de séquences de régions de répétition;

déterminer un ou plusieurs groupes optimaux pour un ensemble de mesures de signaux d'espace de flux parmi la pluralité de mesures de signaux d'espace de flux, dans lequel au moins un des groupes optimaux est associé à une longueur d'homopolymère, dans lequel l'ensemble de mesures de signaux d'espace de flux correspond à un flux donné et à une position dans la séquence de la région de répétition; et

modifier une définition des bases à la position dans la séquence de la région de répétition en longueur de l'homopolymère associée au groupe optimal pour les mesures des signaux d'espace de flux pour le flux donné afin de produire une séquence d'insertion de région de répétition corrigée, corrigeant ainsi une erreur d'insertion ou une erreur de suppression.

9. Système de revendication 8, dans lequel le processeur est en outre configuré pour effectuer une étape comprenant le calcul d'un nombre de répétitions pour la séquence de régions de répétition corrigée.

10. Système selon la revendication 8, dans lequel la détermination d'un ou plusieurs groupes optimaux comprend en outre la génération d'un modèle de mélange de fonctions de densité de probabilité, dans lequel chacune des fonctions de densité de probabilité est associée à un groupe de mesures de signaux d'espace de flux et à un paramètre d'appartenance.

11. Système selon la revendication 10, dans lequel les fonctions de densité de probabilité comprennent des fonctions de densité de probabilité gaussiennes.

12. Système de revendication 10, dans lequel la détermination d'un ou plusieurs groupes optimaux comprend en outre la maximisation d'une probabilité du modèle de mélange pour l'ensemble de mesures de signaux d'espace de flux pour le flux donné en ce qui concerne les paramètres d'appartenance pour former les groupes optimaux.

13. Système de revendication 12, dans lequel la maximisation d'une probabilité du modèle de mélange comprend en outre l'application d'une maximisation d'espérance à un modèle de mélange gaussien.

14. Système de revendication 9, dans lequel le processeur est en outre configuré pour effectuer une étape comprenant l'application d'un appel de variant à la première séquence de bases du flanc gauche et à la deuxième séquence de bases du flanc droit correspondant à la séquence de régions de répétition corrigée pour déterminer un type de variant et un emplacement de variant, et éventuellement pour effectuer une étape comprenant la combinaison des résultats pour le nombre de répétitions pour la séquence de la région de répétition corrigée et le type de variant et l'emplacement du variant pour le flanc gauche et le flanc droit correspondant à la séquence de la région de répétition corrigée.

15. Support de stockage non transitoire lisible par machine comprenant des instructions qui, lorsqu'elles sont exécutées par un processeur, amènent le processeur à effectuer une méthode d'analyse de séquence d'acides nucléiques selon l'une des réclamations 1 à 7.

FIG. 1

FIG. 2

EP 3 539 039 B1

FIG. 3

EP 3 539 039 B1

ALIGNED SEQUENCE READS

REFERENCE
SEQUENCE
→ ALIGN FLANKS OF SEQUENCE
READS AND REFERENCE — 402 → 420

FLOW SPACE
SIGNAL
MEASUREMENTS
→ RECEIVE FLOW SPACE SIGNAL
MEASUREMENTS IN REPEAT
REGION — 404

DETERMINE OPTIMUM CLUSTERS — 406

MODIFY BASE CALL IN REPEAT
REGION SEQUENCE TO
OPTIMUM HOMOPOLYMER LENGTH — 408

CALCULATE LENGTH OF MODIFIED
REPEAT REGION SEQUENCE — 410 → 422

FIG. 4

FIG. 5

FIG. 6

EP 3 539 039 B1

FIG. 7A

FIG. 7B

FIG. 8A

FIG. 8B

EP 3 539 039 B1

FIG. 9A

FIG. 9B

FIG. 10A

FIG. 10B

420

EXTRACT LEFT AND
RIGHT FLANK SEQUENCES —1102

DETECT VARIANTS IN
FLANK SEQUENCES —1104

422—▶ COMBINE VARIANT AND
REPEAT REGION RESULTS —1106

FIG. 11

Left
Flank          STR          Right
                            Flank

1201

FIG. 12

FIG. 13

EP 3 539 039 B1

200

202

Fluidic
Delivery
and
Control
Unit

206
Signal
Detection
Unit

208

Data
Acquisition,
Analysis
and Control
Unit

204
Sample Processing Unit

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62420022 A
- US 20130345066 A
- US 20120173159 A
- US 20120197623 A
- US 20090127589
- US 20090026082 A
- US 20090325145

**Non-patent literature cited in the description**

- **SMITH** ; **WATERMAN**. *Journal of Molecular Biology*, 1981, vol. 147 (10), 195-197

- **DEMPSTER, A.P** ; **LAIRD, N.M** ; **RUBIN, D.B**. Maximum Likelihood from Incomplete Data via the EM Algorithm. *Journal of the Royal Statistical Society*, 1977, vol. 39 (1), 1-38